# EUROPÄISCHE PATENTSCHRIFT

(11) **EP 1 323 830 B1**
(45) Veröffentlichungstag und Bekanntmachung des Hinweises auf die Patenterteilung: **26.04.2006**
(21) Anmeldenummer: 02026848.8
(22) Anmeldetag: 29.11.2002
(51) Int. Cl.: C12P 17/04, C12P 17/06, C12P 17/18, C12N 9/88

(54) **Verfahren zur Herstellung von heterocyclischen (R)-und (S)-Cyanhydrinen**
Process for the preparation of heterocyclic (R)- et (S)-cyanohydrines
Procédé pour la préparation de (R)- et (S)-cyanohydrines hétérocycliques

(30) Priorität: 27.12.2001 AT 20332001
(43) Veröffentlichungstag der Anmeldung: 02.07.2003
(73) Patentinhaber: DSM Fine Chemicals Austria Nfg GmbH & Co KG, 4021 Linz (AT)
(72) Erfinder: Pöchlauer, Peter, 4040 Linz (AT); Skranc, Wolfgang, 1220 Wien (AT); Mayrhofer, Herbert, 4210 Engerwitzdorf (AT); Wirth, Irma, 4470 Enns (AT); Neuhofer, Rudolf, 4210 Mittertreffling (AT); Griengl, Herfried, 8047 Graz (AT); Fechter, Martin, 8044 Graz (AT)
(74) Vertreter: Lindinger, Ingrid

(56) Entgegenhaltungen:
- WO-A-99/63104
- GREGORY R J H ET AL: "Bicyclo[3.2.0]hept-2-en-6-one cyanohydrins: preparations by chemical hydrocyanation, and enantio- and diastereoselective biotransformation by the hydroxynitrile lyase from Prunus amygdalus in the form of almond meal" TETRAHEDRON LETTERS, ELSEVIER SCIENCE PUBLISHERS, AMSTERDAM, NL, Bd. 40, Nr. 41, 8. Oktober 1999 (1999-10-08), Seiten 7407-7411, XP004178694 ISSN: 0040-4039
- BIANCHI P ET AL: "On the selectivity of oxynitrilases towards alpha-oxygenated aldehydes" TETRAHEDRON, ELSEVIER SCIENCE PUBLISHERS, AMSTERDAM, NL, Bd. 57, Nr. 11, 10. März 2001 (2001-03-10), Seiten 2213-2220, XP004230759 ISSN: 0040-4020
- GUOQIANG L ET AL: "Enzymatic Synthesis of (R)-Cyanohydrins by Three (R)-Oxynitrilase Sources in Micro-aqueous Organic Medium" TETRAHEDRON, ELSEVIER SCIENCE PUBLISHERS, AMSTERDAM, NL, Bd. 55, Nr. 12, 19. März 1999 (1999-03-19), Seiten 3531-3540, XP004158759 ISSN: 0040-4020

## Beschreibung

Die Erfindung betrifft ein enzymkatalysiertes Verfahren zur Herstellung von enantiomerenangereicherten heterocyclischen (R)- und (S)-Cyanhydrinen unter Verwendung von (R)- oder (S)-Hydroxynitrillyase (HNL) aus den korrespondierenden heterocyclischen Ketonen sowie deren weitere Umsetzung zu den korrespondierenden Säuren, Estern oder Amiden.

Cyanhydrine sind zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, von Bedeutung.

Die Herstellung dieser Cyanhydrine erfolgt durch Addition von Blausäure an die Carbonylgruppe eines Ketons oder Aldehyds.
Aus der Literatur sind bereits mehrere Verfahrensvarianten bekannt, die die Herstellung von (R)- und/oder (S)-Cyanhydrinen aus aliphatischen, aromatischen oder heteroaromatischen Aldehyden oder auch aus aliphatischen oder aromatischen Ketonen beschreiben.
So wird in EP-A-0 326 063 ein enzymatisches Verfahren zur Herstellung von optisch aktiven (R)- oder (S)-Cyanhydrinen durch Umsetzung von aliphatischen, aromatischen oder heteroaromatischen Aldehyden oder Ketonen mit Blausäure in Gegenwart von (R)-Oxynitrilase (EC 4.1.2.10) aus Prunus amygdalus oder Oxynitrilase (EC 4.1.2.11) aus Sorghum bicolor beansprucht, Beispiele für Ketone, insbesondere von heterocyclischen Ketonen, sind jedoch nicht beschrieben.
EP 0 632 130 beschreibt weiters ein Verfahren, bei welchem aliphatische Aldehyde oder unsymmetrische aliphatische Ketone mit Blausäure und Oxynitrilase aus Hevea brasiliensis stereospezifisch zu (S)-Cyanhydrinen umgesetzt werden. Heterocyclische Ketone sind nicht enthalten.

In EP 0 927 766 ist ein enzymatisches Verfahren zur Herstellung von optisch aktiven (S)-Cyanhydrinen aus aliphatischen, aromatischen oder heteroaromatischen Aldehyden oder Ketonen in Emulsion beschrieben. Als heteroaromatisches Keton wird dabei lediglich Indolylaceton angeführt. Ketone, deren Ketogruppe Bestandteil des Heterocyclus ist, sind aus EP 0 927 766 nicht zu entnehmen.
Cyanhydrine aus heterocyclischen Ketonen, deren Ketogruppe Bestandteil des Heterocyclus ist, wie etwa 7-Oxabicyclo[2.2.1]hept-2-en-derivate, wurden bisher beispielsweise durch Znl₂-katalysierte Diels-Alder-Addition von Furan und Acetoxyacylnitril, wie in Helv. Chim. Acta (1984), 67(6), 1612 - 1615 beschrieben, hergestellt. Optisch aktive Cyanhydrine wurden dabei mittels Racemattrennung über einen Cyanhydrin-Brucin-Komplex erhalten. Eine weitere Möglichkeit stellt die Herstellung von Cyanofuranonen, beispielsweise aus MeCH(OH)CO₂Et und Crotonnitril in THF und in Gegenwart von NaH, wie aus US 4,208,338 bekannt, dar.

Aufgabe der Erfindung war es, ein Verfahren zu finden, das die Herstellung von enantiomerenangereicherten heterocyclischen (R)- und (S)-Cyanhydrinen aus den korrespondierenden heterocyclischen Ketonen auf einfache Weise, in hohen Ausbeuten und mit hoher Enantiomerenreinheit ermöglicht.

Unerwarteterweise konnte diese Aufgabe durch die (R)- oder (S)-HNL-katalysierte Umsetzung von heterocyclischen Ketonen gelöst werden.

Gegenstand der vorliegenden Erfindung ist demnach ein Verfahren zur Herstellung von enantiomerenangereicherten heterocyclischen (R)- und (S)-Cyanhydrinen der Formel (I), 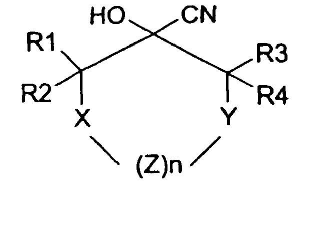 in der R1, R2, R3, R4 unabhängig voneinander H, einen gegebenenfalls ein- oder mehrfach durch unter den Reaktionsbedingungen inerte Substituenten substituierten, linearen, verzweigten oder cyclischen C₁-C₂₄-Alkyl-, Alkenyl- oder Alkinylrest, wobei ein oder mehrere C-Atome in der Kette durch ein Sauerstoffatom, ein Stickstoffatom, ein Schwefelatom, eine SO- oder eine SO₂-Gruppe ersetzt sein können, einen gegebenenfalls ein- oder mehrfach durch unter den Reaktionsbedingungen inerte Substituenten substituierten Aryl- oder Heteroarylrest oder heterocyclischen Rest oder Halogen, Hydroxy, NR5R6, Acetyl, Oxo, C₁-C₆-Carbalkoxy, C₁-C₆-Carbalkoxyamino, COOR7, Cyano, Amid, Benzoylamino oder NO₂ bedeuten,
R5 und R6 unabhängig voneinander H, linearer, verzweigter oder cyclischer C₁-C₆₋Alkylrest, Phenylrest, Benzylrest, COOR7 sein können oder gemeinsam einen C₂-C₈₋Alkylen- oder -Heteroalkylenrest bilden,
R7 gleich H oder C₁-C₆-Alkyl bedeutet,
X und Y unabhängig voneinander ein gegebenenfalls ein- oder zweifach durch unter den Reaktionsbedingungen inerte Substituenten substituiertes C-Atom oder ein Rest aus der Gruppe N, O, S, oder NR5R6, wobei R5 und R6 wie oben definiert sind, SO oder SO₂ sein kann,
n gleich 0, 1, 2 oder 3 ist,
Z ein gegebenenfalls ein- oder zweifach durch unter den Reaktionsbedingungen inerte Substituenten substituiertes C-Atom, oder ein Rest aus der Gruppe N, O, S, oder NR5R6, wobei R5 und R6 wie oben definiert sind, SO oder SO₂ sein kann,
und mindestens einer der Reste X, Y und Z kein C-Atom ist,
wobei die Verbindungen der Formel (I), in Abhängigkeit von der Ringgröße, eine oder mehrere Doppelbindungen im Ring aufweisen können, mit der Maßgabe, dass im 5-er Ring die Doppelbindung nicht in Konjugation zur -C(OH)CN-Gruppe steht,
und/oder ein- oder mehrfach durch 5-er, 6-er oder 7-er Ringe mit 0 bis 3 Heteroatomen anelliert sein können,
und/oder durch einen linearen oder verzweigten C₁-C₆-Alkylenrest, der in der Alkylkette durch ein oder mehrere Heteroatome unterbrochen sein kann und/oder eine Doppelbindung aufweisen kann, überbrückt sein können,
das dadurch gekennzeichnet ist, dass Ketone der Formel (II), 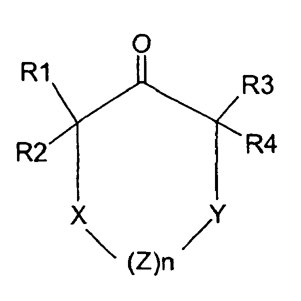 in der R1, R2, R3, R4, X, Y, Z und n obige Bedeutung haben, in Gegenwart eines Cyanidgruppendonors mit einer (R)- oder (S)-Hydroxynitrillyase im organischen, wässrigen oder Zweiphasensystem oder in Emulsion zu den gewünschten (R)- oder (S)-Cyanhydrinen umgesetzt werden.

In der Formel (I) und in der Formel (II) bedeuten R1, R2, R3, R4 unabhängig voneinander H oder einen gegebenenfalls ein- oder mehrfach durch unter den Reaktionsbedingungen inerte Substituenten substituierten, linearen, verzweigten oder cyclischen C₁-C₂₄-Alkyl-, Alkenyl- oder Alkinylrest.
Unter Alkyl, Alkenyl oder Alkinyl sind dabei gesättigte bzw. ein- oder mehrfach ungesättigte, lineare, verzweigte oder cyclische, primäre, sekundäre oder tertiäre Hydrocarbonreste zu verstehen. Dies sind C₁-C₂₄-Alkyl-, Alkenyl- oder Alkinylreste, wie etwa Methyl, Ethyl, Vinyl, Ethinyl, Propyl, i-Propyl, Allyl, Propenyl, 1-Methylcyclopropenyl, Butyl, i-Butyl, t-Butyl, 1,3-Butadienyl, 2-Methyl-1,3-butadienyl, Pentyl, Cyclopentyl, i-Pentyl, neo-Pentyl, 2-Pentinyl, 1,3-Pentadiinyl, Hexyl, i-Hexyl, 1,2-Hexadienyl, Cyclohexyl, Cyclohexylmethyl, 3-Methylpentyl, 2,2-Dimethylbutyl, 2,3-Dimethylbutyl, Octyl, i-Octyl, Cyclooctyl, 2,6-Dimethyloctan, Decyl, Cyclodecyl, Dodecyl, Cyclododecyl, 2,6,10-Trimethyldodecanyl, u.s.w.
Bevorzugt sind dabei C₁-C₁₂-Alkylreste sowie C₂-C₁₂-Alkenyl- oder Alkinylreste, und besonders bevorzugt C₂-C₈-Alkyl-, Alkenyl- oder Alkinylreste.

Bei diesen Resten kann ein oder mehrere C-Atome in der Kette durch ein Sauerstoffatom, ein Stickstoffatom, ein Schwefelatom oder eine SO- oder SO₂-Gruppe ersetzt sein, sodass Ether, Amide, Amine, Imine, Thioether, Sulfoxide und Sulfonyle erhalten werden.
Beispiele dafür sind 2-Methoxypropyl, 2-Methoxybutyl, Oxiranyl, Tetrahydrofuryl, Dioxanyl, 2-Ethoxymethyl, 2-Propoxymethyl, N',N'-Dimethylhydrazino, Ethylthiomethyl, 1,1-Dioxotetrahydrothiophenyl, Methylsufinylmethyl, Methylsulfonylmethyl, Thiiranyl u.s.w.

Als Substituenten, die unter den Reaktionsbedingungen inert sind, eignen sich beispielsweise folgende Gruppen:
C₁-C₂₀-Alkoxy- oder Alkylalkoxy- oder Aryloxygruppen, wie etwa Methoxy-, Ethoxy-, Butoxy-, Hexoxy- Methoxymethyl-, Methoxyethyl-, Ethoxymetyl-, Ethoxyethyl-, Phenyloxy-, u.s.w.;
Nitro-, Halogen-, Hydroxyl-, Oxo-, CN-, CONH₂-, Carboxyl-, Carbonsäureester oder Carbonsäureamide, primäre, sekundäre oder tertiäre Amino-Gruppen, SO₃H-Gruppen, gegebenenfalls ein- oder mehrfach durch Halogen, Hydroxy, C₁-C₄-Alkyl oder Alkoxy substituiertes Phenyl u.s.w.
Bevorzugte Substituenten sind C₁-C₆-Alkoxy, C₁-C₁₂-Alkylalkoxy, C₁-C₂₀-Aryloxy, Halogen, Hydroxy, Oxo, Carboxyl, gegebenenfalls ein- oder zweifach durch Halogen, Hydroxy, C₁-C₄-Alkyl oder C₁-C₄-Alkoxy substituiertes Phenyl.
R1, R2, R3, R4 können aber auch einen gegebenenfalls ein- oder mehrfach durch unter den Reaktionsbedingungen inerte Substituenten substituierten Aryl- oder Heteroarylrest oder heterocyclischen Rest bedeuten.
Unter Aryl sind bevorzugt C₆-C₂₀-Arylgruppen zu verstehen, wie etwa Phenyl, Biphenyl, Naphthyl, Indenyl, Fluorenyl u.s.w.
Die Arylgruppe kann dabei gegebenenfalls durch oben angeführte unter den Reaktionsbedingungen inerte Substituenten ein- oder mehrfach substituiert sein.

Unter Heteroaryl oder Heterocyclus sind cyclische Reste zu verstehen, die zumindest ein O-, S- oder N-Atom im Ring enthalten. Dies sind beispielsweise Furyl, Thiophenyl, Pyridyl, Pyrimidyl, Imidazolyl, Tetrazolyl, Pyrazinyl, Benzofuranyl, Chinolyl, Isochinolyl, Isobenzofuryl, Pyrazolyl, Indolyl, Isoindolyl, Benzoimidazolyl, Purinyl, Carbazolyl, Oxazolyl, Isoxazolyl, Pyrrolyl, Chinazolinyl, Pyridazinyl, Phthalazinyl, Morpholinyl u.s.w.
Funktionelle O- oder N-Gruppen können dabei nötigenfalls geschützt werden.
Die Heteroarylgruppe bzw. der Heterocyclus können dabei gegebenenfalls ein- oder mehrfach durch die bereits oben angeführten Substituenten substituiert sein.
Weiters können R1, R2, R3, R4 auch Halogen, wie etwa Fluor oder Chlor, Bor, Hydroxy, NR5R6, Acetyl, Oxo, C₁-C₆-Carbalkoxy, COOR7, Cyano, NO₂, Amid oder Benzoylamino, bedeuten.
R5 und R6 bedeuten dabei unabhängig voneinander H, einen linearen, verzweigten oder cyclischen C₁-C₆-Alkylrest, Phenylrest, Benzylrest oder COOR7 oder bilden gemeinsam einen C₂-C₈-Alkylen- oder -Heteroalkylenrest.
R7 bedeutet H oder C₁-C₆-Alkyl.

Bevorzugt bedeuten R1, R2, R3 und R4 unabhängig voneinander H, einen gesättigten oder einfach ungesättigten, linearen, verzweigten oder cyclischen C₁-C₁₂₋Alkylrest, in der ein C-Atom in der Kette durch ein Sauerstoffatom, ein Stickstoffatom, oder ein Schwefelatom ersetzt sein kann, einen Phenyl, Biphenyl oder Naphthylrest, oder Halogen, Hydroxy, Oxo, C₁-C₆-Carbalkoxy, C₁-C₆-Carbalkoxyamino, NR5R6, Acetyl, COOR7, Cyano, Amid, Benzoylamino oder NO₂,
R5 und R6 unabhängig voneinander H oder einen linearen, cyclischen oder verzweigten C₁-C₆-Alkylrest sein können oder gemeinsam einen C₂-C₈-Alkylenrest bilden,
und R7 gleich H oder C₁-C₆-Alkyl.

n ist gleich 0,1,2 oder 3, wodurch 5-er, 6-er, 7-er oder 8-er Ringe erhalten werden. Bevorzugt ist n gleich 0, 1 oder 2 und besonders bevorzugt ist n gleich 0 oder 1.

X, Y und Z können unabhängig voneinander ein gegebenenfalls ein- oder zweifach durch unter den Reaktionsbedingungen inerte Substituenten substituiertes C-Atom sein, oder einen Rest aus der Gruppe N, O, S, oder NR5R6, wobei R5 und R6 wie oben definiert sind, SO oder SO₂ bedeuten, wobei mindestens einer der Reste X, Y und Z kein C-Atom ist. Bevorzugt bedeutet nur einer oder zwei der Reste X, Y und Z, besonders bevorzugt nur einer der Reste X, Y und Z, einen Rest aus der Gruppe N, O, S, NR5R6, SO oder SO₂.
Als Substituenten, die unter den Reaktionsbedingungen inert sind, eignen sich wiederum die bereits oben angeführten Substituenten.

Bevorzugt bedeuten X, Y und Z ein gegebenenfalls ein- oder zweifach durch C₁-C₆₋Alkyl, C₁-C₆-Alkoxy, C₁-C₁₂-Alkylalkoxy, C₁-C₂₀-Aryloxy, Halogen, Hydroxy, Oxo, Carboxyl, gegebenenfalls ein- oder zweifach durch Halogen, Hydroxy, C₁-C₄-Alkyl oder C₁-C₄-Alkoxy substituiertes Phenyl substituiertes C-Atom, oder einen Rest aus der Gruppe N, O, S oder NR5R6, bedeuten, wobei einer oder zwei der Reste X, Y oder Z kein C-Atom ist.

Die Verbindungen der Formel (I) und der Formel (II) können dabei in Abhängigkeit von der Ringgröße eine oder mehrere Doppelbindungen im Ring aufweisen, wobei im 5-er Ring die Doppelbindung nicht in Konjugation zur -C(OH)CN-Gruppe stehen darf.
Bevorzugt enthalten 5-er Ringe keine Doppelbindung im Ring. 6-er Ringe enthalten bevorzugt keine oder höchsten eine Doppelbindung im Ring, während in 7-er und 8-er Ringen keine bis zu zwei Doppelbindungen bevorzugt sind.

Die Verbindungen der Formel (I) und der Formel (II) können auch, wiederum in Abhängigkeit von der Ringgröße, ein- oder mehrfach durch 5-er, 6-er oder 7-er Ringe mit 0 bis 3, bevorzugt mit 0 bis 1 Heteroatomen, anelliert sein. Bevorzugt sind die Verbindungen der Formel (I) nicht anelliert oder durch einen 5- oder 6-gliedrigen Ring anelliert.

Weiters können die Verbindungen der Formel (I) und der Formel (II) durch einen linearen oder verzweigten C₁-C₆-Alkylenrest überbrückt sein. Die Alkylenkette kann dabei auch eine Doppelbindung aufweisen und/oder durch ein oder mehrere Heteroatome unterbrochen sein. Bevorzugt ist die Kette durch kein oder höchstens ein Heteroatom unterbrochen.

Bei dem erfindungsgemäßen Verfahren werden Ketone der Formel (II), die zum Teil käuflich erwerbbar sind, oder beispielsweise analog J. Org. Chem., 1970, 35, 898 - 902; oder laut Literatur Synthesis, 1978, 368 - 370, hergestellt werden können, zu enantiomerenangereicherten heterocyclischen (R)- und (S)-Cyanhydrinen umgesetzt.

Die entsprechenden Ketone der Formel (II) werden dabei in Gegenwart eines Cyanidgruppendonors mit einer (R)- oder (S)-Hydroxynitrillyase umgesetzt.

Als Cyanidgruppendonor kommen Blausäure, Alkali-Cyanide oder Cyanhydrine der allgemeinen Formel (III)

R8R9C(OH)(CN)

in Betracht. In der Formel (III) bedeuten R8 und R9 unabhängig voneinander Wasserstoff oder eine unsubstituierte Kohlenwasserstoffgruppe, oder R8 und R9 gemeinsam eine Alkylengruppe mit 4 oder 5 C-Atomen, wobei R8 und R9 nicht gleichzeitig Wasserstoff bedeuten. Die Kohlenwasserstoffgruppen sind aliphatische oder aromatische, bevorzugt aliphatische, Gruppen. Bevorzugt bedeuten R8 und R9 Alkylgruppen mit 1 - 6 C-Atomen, ganz bevorzugt ist Acetocyanhydrin als Cyanidgruppendonor der Formel (III).
Die Herstellung des Cyanidgruppendonors kann nach bekannten Verfahren erfolgen. Cyanhydrine, insbesondere Acetocyanhydrin, sind auch käuflich zu erwerben.

Bevorzugt wird Blausäure, KCN, NaCN, oder Acetocyanhydrin, besonders bevorzugt Blausäure als Cyanidgruppendonor eingesetzt.

Die Blausäure kann dabei auch erst kurz vor der Reaktion aus einem ihrer Salze, wie etwa NaCN oder KCN, freigesetzt und in Substanz oder in gelöster Form dem Reaktionsgemisch zugegeben werden.

Der Cyanidgruppendonor wird dabei in einem molaren Verhältnis zur Verbindung der Formel (II) von 0,5:1 bis 7:1, bevorzugt von 0,8:1 bis 6:1 und besonders bevorzugt von 1:1 bis 5:1 eingesetzt.

Die Umsetzung kann im organischen, wässrigen oder Zweiphasensystem oder in Emulsion durchgeführt werden.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden t-Butylmethylether, Diisopropylether, Dibutylether und Ethylacetat oder deren Gemische eingesetzt.
Bei der enantioselektiven Umsetzung wird als wässriges System eine wässrige, die entsprechende HNL-enthaltende Lösung oder Pufferlösung verwendet. Beispiele dafür sind Acetatpuffer-, Boratpuffer-, Phthalatpuffer-, Citratpuffer- oder Phosphatpufferlösung oder Gemische davon.

Die HNLs können dabei entweder als solche oder immobilisiert im organischen Verdünnungsmittel vorliegen, die Umsetzung kann jedoch auch in einem Zweiphasensystem oder in Emulsion mit nicht-immobilisierter HNL erfolgen.

Als HNLs eignen sich sowohl native als auch rekombinante (R)- und (S)-HNLs.

Als (S)-Hydroxynitrillyase kommen native (S)-Hydroxynitrillyasen, z. B. aus Maniok und Hevea brasiliensis, sowie rekombinante (S)-HNL in Frage. Bevorzugt wird als native HNL jene aus Hevea brasiliensis verwendet. Geeignete rekombinante (S)-HNL wird beispielsweise aus gentechnisch modifizierten Mikroorganismen, wie etwa Pichia pastoris; E. coli oder Saccharomyces cerevisiae, erhalten.
Bevorzugt wird rekombinante (S)-HNL aus Pichia pastoris oder E. coli eingesetzt.

Als (R)-HNL kommen beispielsweise (R)-Hydroxynitrillyasen aus Prunus amygdalus, Prunus laurocerasus oder Prunus serotina, oder rekombinante (R)-HNL in Frage. Bevorzugt wird (R)-Hydroxynitrillyase aus Prunus amygdalus oder eine rekombinante (R)-HNL verwendet.

Geeignete (R)- und (S)-HNLs sind beispielsweise aus WO 97/03204, EP 0 969 095; EP 0 951 561, EP 0 927 766, EP 0 632 130, EP 0547 655, EP 0 326 063, WO 01/44487 u.s.w. bekannt.

Pro g Keton werden etwa 0,1 bis 20 g Verdünnungsmittel und 10 bis 50 000 IU, bevorzugt 1 000 bis 40 000 IU, Aktivität Hydroxynitrillyase zugesetzt.

Die Reaktionsmischung wird bei Temperaturen von etwa - 10 °C bis zur Deaktivierungstemperatur der Hydroxynitrillyase, bevorzugt bei - 5 bis + 30 °C geschüttelt oder gerührt, oder bei Umsetzung in Emulsion bei Temperaturen von 0 °C bis etwa + 30 °C derart gerührt, dass eine Emulsion entsteht.

Zur Aufarbeitung des Reaktionsgemisches und zur Isolierung des gebildeten Cyanhydrines werden im Falle der Reaktion in Emulsion übliche Techniken, welche zunächst die Emulsion brechen, wie etwa Filtration, Zentrifugation oder Koaleszierung eingesetzt. Anschließend werden die sich bildenden Phasen gegebenenfalls unter Zugabe von Demulgatoren aufgetrennt und die produkthaltige Phase aufgearbeitet.

Zur Gewinnung des entsprechenden Cyanhydrines werden dabei je nach Endprodukt bekannte Techniken wie Destillation, Extraktion oder Kristallisation angewandt. Die so gewonnenen Cyanhydrine können gegebenenfalls durch Zugabe einer Säure vor der Weiterverarbeitung stabilisiert werden.

Bei der Extraktion werden dabei organische Lösungsmittel, die mit Wasser nicht mischbar sind, wie etwa aliphatische oder aromatische gegebenenfalls halogenierte Kohlenwasserstoffe, z. B. Pentan, Hexan, Benzol, Toluol, Methylenchlorid, Chloroform, Chlorbenzole, Ether wie etwa t-Butylmethylether, Diethylether, Diisopropylether oder Ester, beispielsweise Essigsäureethylester oder Mischungen solcher Lösungsmittel, verwendet.
Sollte die Reinheit des extrahierten Produktes nicht ausreichend sein, kann eine Reinigungsoperation angeschlossen werden. Die Reinigung kann durch eine bekannte Methode erfolgen und gelingt am besten chromatographisch.

Die erfindungsgemäß hergestellten (R)- oder (S)-Cyanhydrine der Formel (I) werden dabei in hohen Ausbeuten und mit einer hohen optischen Reinheit erhalten.

Die Cyanhydrine der Formel (I) können gewünschtenfalls noch weiter verarbeitet werden, wodurch die korrespondierenden Hydroxycarbonsäuren, deren Ester, sowie korrespondierende Ether oder Amide erhalten werden können.
Das entsprechende (R)- und (S)-Cyanhydrin der Formel (I) kann beispielsweise nach Extraktion oder gegebenenfalls nach Abfiltrieren des Enzyms und Abdestillieren des Lösungsmittels ohne weitere Reinigung analog dem Stand der Technik, beispielsweise wie in Angew. Chem. 1994, 106, 1615 oder in Tetrahedron Letters 1990, Vol. 31, No. 9, 1249 - 1252 beschrieben, mit konzentrierter Salzsäure hydrolysiert werden. Für die Hydrolyse können auch andere geeignete Säuren wie etwa H₂SO₄ verwendet werden, bevorzugt wird jedoch HCl eingesetzt.

Die so erhaltenen (R)- und (S)-α-Hydroxycarbonsäuren können sodann gegebenenfalls durch Umkristallisation, wie etwa in EP 1 148 042 beschrieben, aufgereinigt werden.

Die (R)- und (S)-α-Hydroxycarbonsäuren können wiederum durch Umsetzung mit einer Alkyliodidverbindung, beispielsweise mit Methyliodid, in Anwesenheit von Ag-Salzen, wie etwa in Bull. Chem. Soc. Jpn., 1967, 40, 373 - 378 beschrieben, in den korrespondierenden Ether überführt werden.

Die Herstellung von korrespondierenden Amiden aus Cyanhydrinen der Formel (I) kann beispielsweise durch partielle Hydrolyse mit HBF₄ in CH₂Cl₂ bei 0 °C, wie etwa in Tetrahedron Asymmetry, 1997, 8, 3503 - 3511 beschrieben, erfolgen.

Korrespondierende Ester werden beispielsweise durch Acylierung mit Acetylchlorid und Pyridin in CH₂Cl₂, wie etwa in Tetrahedron, 1998, 54, 14477 - 14486 beschrieben, hergestellt werden.

Ein weiterer Gegenstand der Erfindung ist demnach die Verwendung der erfindungsgemäß hergestellten (R)- oder (S)-Cyanhydrine der Formel (I) zur Herstellung der korrespondierenden Hydroxycarbonsäuren, Ether, Ester und Amide.

### Beispiel 1: Synthese von (S)-3-Cyano-tetrahydrothiophen-3-ol:

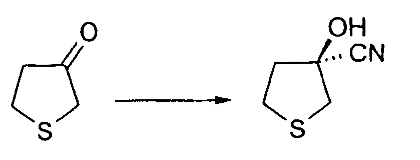

10,2 g (0,1 mol) 4,5-Dihydro-3(2H)-thiophenon wurden in 35 mL t-Butylmethylether gelöst und auf 0 °C gekühlt.
31 mL (S)-HNL von Hevea brasiliensis mit einer Aktivität von 6500 IU/mL wurden mit 24 mL eines 50 mmol K₂HPO₄/Citrat Puffers mit pH 4,00 gemischt und mit 10 % Citronensäure auf pH 4,50 gestellt. Diese wässrige enzymatische Lösung wurde zur organischen Lösung gegeben und 5 Minuten bei 0 °C zu einer Emulsion gerührt. 13,5 g (0,5 mol) HCN wurden daraufhin innerhalb von 40 Minuten der Reaktionslösung unter intensiver Rührung zugegeben. Nach 75 Minuten wurden 100 mL t-Butylmethylether zur Reaktion getropft und weitere 30 Minuten gerührt.

Nach erfolgreicher Phasentrennung wurde die wässrige Phase neuerlich mit 100 mL t-Butylmethylether extrahiert und die vereinigten organischen Phasen über Na₂SO₄ getrocknet, abfiltriert und vom Lösungmittel befreit.

Ausbeute: 10,26 g gelbes Öl (79,4 % Ausbeute, 91,3 % ee)

### Beispiel 2: Synthese von (S)-3-Hydroxy-tetrahydrothiophen-3-carbonsäure:

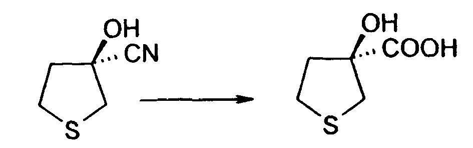

6,0 g (0,046 mol) (S)-3-Cyano tetrahydrothiophen-3-ol, hergestellt nach Beispiel 1, wurden in 15,4 mL konz. HCl suspendiert und auf 70 °C erwärmt. Nach 15 Stunden Rühren bei 70 °C wurde die Lösung abgekühlt, mit wässriger NaOH basisch gestellt und zweimal mit t-Butylmethylether extrahiert. Die gelbe wässrige Phase wurde mit HCl auf pH 1 gestellt und zweimal mit t-Butylmethylether extrahiert. Die vereinigten t-Butylmethylether Phasen wurden mit NaCl Lösung gewaschen, über Na₂SO₄ getrocknet, abfiltriert und vom Lösungmittel befreit.

Ausbeute: 3,85 g beiger Feststoff (55,9 % Ausbeute, 91,3 % ee)

Zwecks Bestimmung der Chiralität der Säure wurde das Salz mit (S)-Phenylethylamin hergestellt und die Absolutkonfiguration der Säure durch Röntgenstruktur bestimmt.

### Beispiel 3: Synthese von (S)-3-Cyanotetrahydrofuran-3-ol:

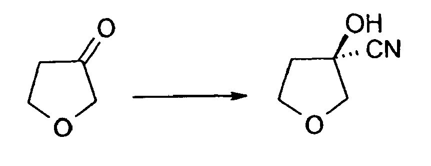

0,52 g (6,05 mmol) 4,5-Dihydro-3(2H)-furanon wurde in 3,5 mL t-Butylmethylether gelöst und auf 0 °C gekühlt.
3,10 mL (R)-HNL von Prunus amygdalus mit einer Aktivität von 2100 IU/mL wurden mit 2,4 mL eines 50 mmol K₂HPO₄/Citrat Puffers mit pH 4,00 gemischt und mit 10 % Citronensäure auf pH 4,50 gestellt. Diese wässrige Phase wurde zur organischen Phase chargiert und 5 Minuten bei 0 °C gerührt. Daraufhin wurden 0,76 g (28 mmol) HCN auf einmal hinzugegeben und 1 h lang gerührt. Die Reaktionslösung wurde dreimal mit t-Butylmethylether extrahiert und die vereinigten organischen Phasen vom Lösungmittel befreit.

Ausbeute: 92 % Umsatz mit 24 % ee, hell gelbes Öl

### Beispiel 4: Synthese von (S)-3-Cyanotetrahydropyran-3-ol

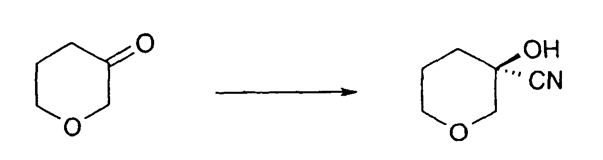

0,200 g (2 mmol) Tetrahydro-4H-pyran-3-on wurden in 3 mL t-Butylmethylether gelöst und auf 0 °C gekühlt.
0,3 mL (S)-HNL von Hevea brasiliensis mit einer Aktivität von 5100 IU/mL wurden mit 3 mL Aqua dest. gemischt und mit 10 % Citronensäure auf pH 4,70 gestellt. Diese wässrige enzymatische Lösung wurde zur organischen Lösung gegeben und 15 Minuten bei 0 °C zu einer Emulsion gerührt. 0,3 mL (8,8 mmol) HCN wurden daraufhin in die Reaktionslösung gegeben. Nach 60 Minuten Rühren bei 0 °C wurden 3 mL t-Butylmethylether zur Reaktion hinzugefügt und mit 0,5 g Celite® 545 Wasser und Enzym gebunden. Anschließend wurde über Na₂SO₄ getrocknet, abfiltriert und vom Lösungsmittel befreit.
Ausbeute: (98% Umsatz) farbloses Öl an (S)-3-Cyanotetrahydropyran-3-ol mit 44 % ee

Für analytische Zwecke wurde das Cyanhydrin in das entsprechende Acetat verwandelt und als solches charakterisiert.
44 % ee; ¹H NMR: δ (ppm) 4.05 (d, J = 12.1 Hz, 1H; H-2), 3.51 (d, 1H; H-2'), 3.87-3.61 (m, 2H; H-6, H-6'), 2.38 (m, 1H; H-4), 2.15 (m, 1H; H4'), 2.13 (s, 3H; Ac-CH₃), 1.85 (m, 2H; H-5, H-5'); ¹³C NMR: δ (ppm), 168.99 (Ac-C=O), 117.39 (CN), 68.93, 68.09 (C-2, C-6), 32.83, 30.52 (C-4, C-5), 22.03 (Ac-CH₃).

### Beispiel 5: Umsetzung von rac-2-Methyltetrahydrofuran-3-on

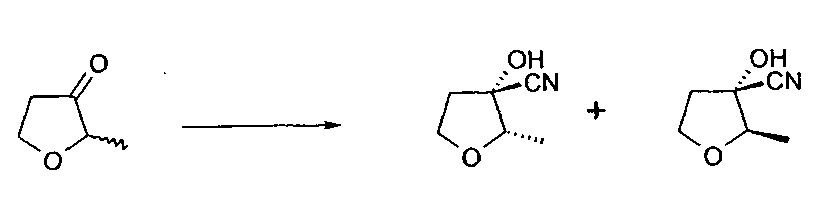

0,200 g (2 mmol) 2-Methyltetrahydrofuran-3-on wurden in 3 mL t-Butylmethylether gelöst und auf 0 °C gekühlt.
1,5 mL (S)-HNL von Hevea brasiliensis mit einer Aktivität von 5100 IU/mL wurden mit 1,5 mL Aqua dest. verdünnt und mit 10 % Citronensäure auf pH 5,20 gestellt. Diese wässrige enzymatische Lösung wurde zur organischen Lösung gegeben und 15 Minuten bei 0 °C zu einer Emulsion gerührt. 0,3 mL (8,8 mmol) HCN wurden daraufhin in die Reaktionslösung gegeben. Nach 60 Minuten Rühren bei 0 °C wurden 3 mL t-Butylmethylether zur Reaktion hinzugefügt und mit 0,5 g Celite® 545 Wasser und Enzym gebunden. Anschließend wurde über Na₂SO₄ getrocknet, abfiltriert und vom Lösungsmittel befreit.
Ausbeute: (99% Umsatz) farbloses Öl an 3-Cyano-2-methyltetrahydrofuran-3-ol

Für analytische Zwecke wurde das Cyanhydrin in das entsprechende Acetat verwandelt und als solches charakterisiert.
(+)-cis Isomer: 27 % de; ¹H NMR: δ (ppm) 4.13-4.04 (m, 1H; H-5), 4.04 (qu, J = 6.3 Hz, 1H; H-2), 3.85 (ddd, , J = 18.0, 8.8, 6.9 Hz 1H; H-5'), 2.71 (ddd, J = 14.0, 8.8, 8.2 Hz 1H; H-4),2.43 (ddd, J = 14.0, 6.9, 3.5 Hz 1H; H4'), 2.15 (s, 3H; Ac-CH₃), 1.50 (d, J = 6.3 Hz, 3H; CH₃); ¹³C NMR: δ (ppm), 169.23 (Ac-C=O), 116.35 (CN), 81.98 (C-5), 78.78 (C-3), 65.87 (C-2), 38.86 (C-4), 20.79 (Ac-CH₃), 17.32 (CH₃).
(+)-trans Isomer: 50 % de; ¹H NMR: δ (ppm) 4.13-4.02 (m, 1H; H-5), 4.11 (qu, J = 6.3 Hz, 1H; H-2), 3.88 (ddd, , J = 17.6, 8.5, 7.3 Hz 1H; H-5'), 2.78 (ddd, J = 14.6, 8.3, 5.4 Hz 1H; H-4),2.51 (ddd, J = 14.6, 8.5, 7.3 Hz 1H; H4'), 2.16 (s, 3H; Ac-CH₃), 1.40 (d, J = 6.3 Hz, 3H; CH₃); ¹³C NMR: δ (ppm), 169.12 (Ac-C=O), 117.05 (CN), 82.09 (C-5), 75.20 (C-3), 65.84 (C-2), 38.70 (C-4), 20.70 (Ac-CH₃), 13.32 (CH₃).

### Beispiel 6: Umsetzung von rac-2-Methyltetrahydrothiophen-3-on

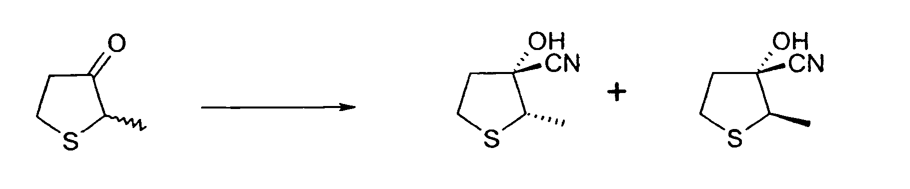

0,232 g (2 mmol) 2-Methyltetrahydrothiophen-3-on wurden in 3 mL t-Butylmethylether gelöst und auf 0 °C gekühlt.
1 mL (S)-HNL von Hevea brasiliensis mit einer Aktivität von 5100 IU/mL wurden mit 2 mL Aqua dest. verdünnt und mit 10 % Citronensäure auf pH 4,40 gestellt. Diese wässrige enzymatische Lösung wurde zur organischen Lösung gegeben und 15 Minuten bei 0°C zu einer Emulsion gerührt. 0,3 mL (8,8 mmol) HCN wurden daraufhin in die Reaktionslösung gegeben. Nach 60 Minuten Rühren bei 0 °C wurden 3 mL t-Butylmethylether zur Reaktion hinzugefügt und mit 0,5 g Celite® 545 Wasser und Enzym gebunden. Anschließend wurde über Na₂SO₄ getrocknet, abfiltriert und vom Lösungsmittel befreit.
Ausbeute: (90%-Umsatz) gelbes Öl an 3-Cyano-2-methyltetrahydrothiophen-3-ol

Für analytische Zwecke wurde das Cyanhydrin in das entsprechende Acetat überführt und als solches charakterisiert.
(+)-cis Isomer: 10 % de; ¹H NMR: : δ (ppm) 3.65 (qu, J = 6.9 Hz, 1H; H-2), 3.12-2.70 (m, 3H; H-4, H-5, H-5'), 2.58-2.37 (m, 1H; H-4'), 2.10 (s, 3H; Ac-CH₃), 1.47 (d, J = 6.9 Hz, 3H; CH₃); ¹³C NMR: δ (ppm), 169.02 (Ac-C=O), 115.54 (CN), 81.38 (C-3), 48.06 (C-2), 38.70 (C-4), 26.95 (C-5), 20.98 (Ac-CH₃), 19.48 (CH₃).
(+)-trans Isomer: 10 % de; ¹H NMR: δ (ppm) 3.85 (qu, J = 6.9 Hz, 1H; H-2), 3.12-2.70 (m, 3H; H-4, H-5, H-5'), 2.58-2.37 (m, 1H; H-4'), 2.14 (s, 3H; Ac-CH₃), 1.34 (d, J = 6.9 Hz, 3H; CH₃); ¹³C NMR: δ (ppm), 168.97 (Ac-C=O), 117.00 (CN), 78.48 (C-3), 48.74 (C-2), 38.66 (C-4), 26.34 (C-5), 20.88 (Ac-CH₃), 15.62 (CH₃).

### Beispiel 7: Umsetzung von rac-5-Methyltetrahydrothiophen-3-on

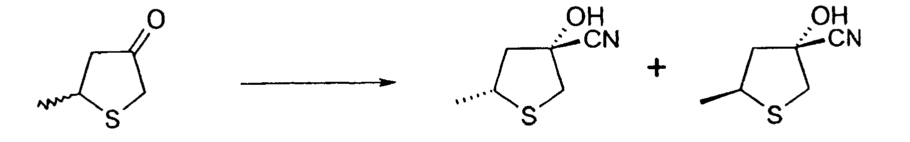

0,232 g (2 mmol) 2-Methyltetrahydrothiophen-3-on wurden in 3 mL t-Butylmethylether gelöst und auf 0 °C gekühlt.
3 mL (R)-HNL von Prunus amygdalus mit einer Aktivität von 250 IU/mL wurden mit 10 % Citronensäure auf pH 4,30 eingestellt. Diese wässrige enzymatische Lösung wurde zur organischen Lösung gegeben und 15 Minuten bei 0 °C zu einer Emulsion gerührt. 0,3 mL (8,8 mmol) HCN wurden daraufhin in die Reaktionslösung gegeben. Nach 60 Minuten Rühren bei 0 °C wurden 3 mL t-Butylmethylether zur Reaktion hinzugefügt und mit 0,5 g Celite® 545 Wasser und Enzym gebunden. Anschließend wurde über Na₂SO₄ getrocknet, abfiltriert und vom Lösungsmittel befreit. Ausbeute: (95% Umsatz) gelbes Öl an 3-Cyano-5-methyltetrahydrothiophen-3-ol

Für analytische Zwecke wurde das Cyanhydrin in das entsprechende Acetat überführt und als solches charakterisiert.
(-)-cis Isomer: 86 % de; ¹H NMR: δ (ppm) 3.77-33.6 (m, 3H; H-5, H-2, H-2'), 2.81 (dd, J = 13.2, 6.6 Hz, 1H; H-4), 2.11 (m, 1H; H-4') 2.13 (s, 3H; Ac-CH₃), 1.41 (d, J = 6.8 Hz, 3H; CH₃); ¹³C NMR: δ (ppm), 168.97 (Ac-C=O), 117.02 (CN), 76.67 (C-3), 49.35 (C-2), 41.24 (C-4), 38.51 (C-5), 22.00 (Ac-CH₃), 20.63 (CH₃).
(-)-trans Isomer: 95 % de; ¹H NMR: δ (ppm) 3.70 (m, 1H; H-5), 3.62 (d, J = 11.9 Hz, 1H; H-2), 3.28 (d, J = 11.9 Hz, 1H; H-2'), 2.88 (dd, J = 13.2, 6.6 Hz, 1H; H-4), 2.12 (dd, J = 13.2 Hz, 1H; H-4') 2.13 (s, 3H; Ac-CH₃), 1.40 (d, J = 6.8 Hz, 3H; CH₃); ¹³C NMR: δ (ppm), 168.99 (Ac-C=O), 117.47 (CN), 77.41 (C-3), 48.47 (C-2), 40.54 (C-4), 38.51 (C-5), 21.99 (Ac-CH₃), 20.89 (CH₃).

## Patentansprüche

1. Verfahren zur Herstellung von enantiomerenangereicherten heterocyclischen (R)- und (S)-Cyanhydrinen der Formel (I), 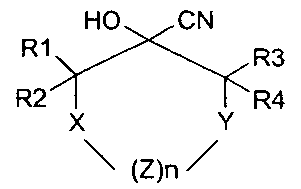 in der R1, R2, R3, R4 unabhängig voneinander H, einen gegebenenfalls ein- oder mehrfach durch unter den Reaktionsbedingungen inerte Substituenten substituierten, linearen, verzweigten oder cyclischen C₁-C₂₄-Alkyl-, Alkenyl- oder Alkinylrest, wobei ein oder mehrere C-Atome in der Kette durch ein Sauerstoffatom, ein Stickstoffatom, ein Schwefelatom, eine SO- oder eine SO₂-Gruppe ersetzt sein können, einen gegebenenfalls ein- oder mehrfach durch unter den Reaktionsbedingungen inerte Substituenten substituierten Aryl- oder Heteroarylrest oder heterocyclischen Rest oder Halogen, Hydroxy, NR5R6, Acetyl, Oxo, C₁-C₆-Carbalkoxy, C₁-C₆₋Carbalkoxyamino, COOR7, Cyano, Amid, Benzoylamino oder NO₂ bedeuten,
R5 und R6 unabhängig voneinander H, linearer, verzweigter oder cyclischer C₁₋C₆-Alkylrest, Phenylrest, Benzylrest, COOR7 sein können oder gemeinsam einen C₂-C₈-Alkylen- oder-Heteroalkylenrest bilden,
R7 gleich H oder C₁-C₆-Alkyl bedeutet,
X und Y unabhängig voneinander ein gegebenenfalls ein- oder zweifach durch unter den Reaktionsbedingungen inerte Substituenten substituiertes C-Atom oder ein Rest aus der Gruppe N, O, S, oder NR5R6, wobei R5 und R6 wie oben definiert sind, SO oder SO₂ sein kann,
n gleich 0, 1, 2 oder 3 ist,
Z ein gegebenenfalls ein- oder zweifach durch unter den Reaktionsbedingungen inerte Substituenten substituiertes C-Atom, oder ein Rest aus der Gruppe N, O, S, oder NR5R6, wobei R5 und R6 wie oben definiert sind, SO oder SO₂ sein kann,
und mindestens einer der Reste X, Y und Z kein C-Atom ist,
wobei die Verbindungen der Formel (I), in Abhängigkeit von der Ringgröße, eine oder mehrere Doppelbindungen im Ring aufweisen können, mit der Maßgabe, dass im 5-er Ring die Doppelbindung nicht in Konjugation zur -C(OH)CN-Gruppe steht,
und/oder ein- oder mehrfach durch 5-er, 6-er oder 7-er Ringe mit 0 bis 3 Heteroatomen anelliert,
und/oder durch einen linearen oder verzweigten C₁-C₆-Alkylenrest, der in der Alkylkette durch ein oder mehrere Heteroatome unterbrochen sein kann und/oder eine Doppelbindung aufweisen kann, überbrückt sein können,
**dadurch gekennzeichnet, dass** Ketone der Formel (II), 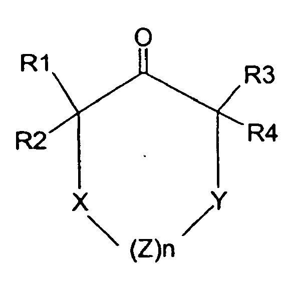 in der R1, R2, R3, R4, X, Y, Z und n obige Bedeutung haben, in Gegenwart eines Cyanidgruppendonors mit einer (R)- oder (S)-Hydroxynitrillyase im organischen, wässrigen oder Zweiphasensystem oder in Emulsion zu den gewünschten (R)- und (S)-Cyanhydrinen umgesetzt werden.

2. Verfahren nach Anspruch 1, **dadurch gekennzeichnet, dass** Ketone der Formel (II) als Edukte eingesetzt werden, in der R1, R2, R3 und R4 unabhängig voneinander H, einen gesättigten oder einfach ungesättigten, linearen, verzweigten oder cyclischen C₁-C₁₂-Alkylrest, in der ein C-Atom in der Kette durch ein Sauerstoffatom, ein Stickstoffatom oder ein Schwefelatom ersetzt sein kann, einen Phenyl, Biphenyl oder Naphthylrest bedeuten, wobei die Reste durch einen oder mehreren Substituenten aus der Gruppe C₁-C₆-Alkyl, C₁-C₆-Alkoxy, C₁-C₁₂-Alkylalkoxy, C₁-C₂₀₋Aryloxy, Halogen, Hydroxy, Oxo, Carboxyl, gegebenenfalls ein- oder zweifach durch Halogen, Hydroxy, C₁-C₄-Alkyl oder C₁-C₄-Alkoxy substituiertes Phenyl substituiert sein können,
oder Halogen, Hydroxy, Oxo, C₁-C₆-Carbalkoxy, C₁-C₆-Carbalkoxyamino, NR5R6, Acetyl, COOR7, Cyano, Amid, Benzoylamino oder NO₂ bedeuten,
R5 und R6 unabhängig voneinander H oder einen linearen, cyclischen oder verzweigten C₁-C₆-Alkylrest sein können oder gemeinsam einen C₂-C₈-Alkylenrest bilden,
R7 gleich H oder C₁-C₆-Alkyl bedeutet,
n gleich 0, 1 oder 2 ist,
X, Y und Z unabhängig voneinander ein gegebenenfalls ein- oder zweifach durch C₁-C₆-Alkyl, C₁-C₆-Alkoxy, C₁-C₁₂-Alkylalkoxy, C₁-C₂₀-Aryloxy Halogen, Hydroxy, Oxo, Carboxyl, gegebenenfalls ein- oder zweifach durch Halogen, Hydroxy, C₁-C₄₋Alkyl oder C₁-C₄-Alkoxy substituiertes Phenyl substituiertes C-Atom, oder einen Rest aus der Gruppe N, O, S oder NR5R6, bedeuten, wobei einer oder zwei der Reste X, Y oder Z kein C-Atom ist,
wobei die Ketone der Formel (II) bei n = 0 keine Doppelbindung, bei n = 1 keine oder eine Doppelbindung und bei n = 2 keine bis zu zwei Doppelbindungen enthalten und/oder gegebenenfalls durch einen 5-er oder 6-er Ring mit 0 bis 1 Heteroatomen anelliert sind,
und/oder durch einen linearen oder verzeigten C₁-C₆-Alkylenrest, der gegebenenfalls eine Doppelbindung aufweist, überbrückt sein können.

3. Verfahren nach Anspruch 1, **dadurch gekennzeichnet, dass** als organisches Verdünnungsmittel für die Reaktion im organischen System mit Wasser nicht oder geringfügig mischbare aliphatische oder aromatische Kohlenwasserstoffe, die gegebenenfalls halogeniert sind, Alkohole, Ether oder Ester oder Gemische eingesetzt werden.

4. Verfahren nach Anspruch 1, **dadurch gekennzeichnet, dass** die Umsetzung in Emulsion erfolgt.

5. Verfahren nach Anspruch 1, **dadurch gekennzeichnet, dass** die enantioselektive Umsetzung im wässrigen System durchgeführt wird, wobei eine die entsprechende Hydroxynitrillyase enthaltende Lösung oder Acetatpuffer-, Boratpuffer-, Phthalatpuffer-, Citratpuffer- oder Phosphatpufferlösung oder Gemische dieser Pufferlösungen Reaktionsmedium dient.

6. Verfahren nach Anspruch 1, **dadurch gekennzeichnet, dass** als Hydroxynitrillyase native oder rekombinante (R)- und (S)-Hydroxynitrillyasen eingesetzt werden, die entweder als solche oder immobilisiert vorliegen.

7. Verfahren nach Anspruch 6, **dadurch gekennzeichnet, dass** als Hydroxynitrillyase native (S)-Hydroxynitrillyasen aus Maniok und Hevea brasiliensis, rekombinante (S)-Hydroxynitrillyase aus gentechnisch modifizierten Mikroorganismen aus der Gruppe Pichia pastoris, E. coli oder Saccharomyces cerevisiae, native (R)-Hydroxynitrillyasen aus Prunus amygdalus, Prunus laurocerasus oder Prunus serotina oder rekombinante (R)-Hydroxynitrillyasen eingesetzt werden.

8. Verfahren nach Anspruch 1, **dadurch gekennzeichnet, dass** als Cyanidgruppendonor Blausäure, Alkalicyanide oder Cyanhydrine der allgemeinen Formel (III),
R7R8C(OH)(CN)
in der R7 und R8 unabhängig voneinander Wasserstoff oder eine unsubstituierte Kohlenwasserstoffgruppe bedeuten, oder R7 und R8 gemeinsam eine Alkylengruppe mit 4 oder 5 C-Atomen, wobei R7 und R8 nicht gleichzeitig Wasserstoff bedeuten, bilden.

9. Verfahren nach Anspruch 1, desweiteren **dadurch gekennzeichnet, dass** die (R)- oder (S)-Cynahydrine der Formel I zur Herstellung der korrespondierenden Hydroxycarbonsäuren, Ether, Ester oder Amide verwendet werden.

## Claims

1. Process for preparing enantiomer-enriched heterocyclic (R)- und (S)-cyanohydrins of the formula (I), 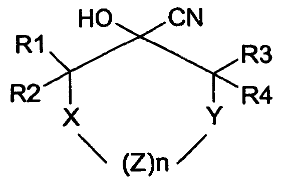 where R1, R2, R3, R4 independently of one another are H, an unbranched, branched or cyclic C₁-C₂₄-alkyl, alkenyl or alkynyl radical which can be unsubstituted, mono substituted or polysubstituted by substituents inert under the reaction conditions, where one or more carbon atoms in the chain can be replaced by an oxygen atom, a nitrogen atom, a sulphur atom, an SO or an SO₂ group, an aryl or heteroaryl or heterocyclic radical which can be unsubstituted, monosubstituted or polysubstituted by substituents inert under the reaction conditions, or halogen, hydroxyl, NR5R6, acetyl, oxo, C₁-C₆-carbalkoxy, C₁-C₆₋carbalkoxyamino, COOR7, cyano, amide, benzoylamino or NO₂,
R5 and R6 independently of one another can be H, unbranched, branched or cyclic C₁-C₆-alkyl radical, phenyl radical, benzyl radical, COOR7, or together form a C₂-C₈-alkylene or -heteroalkylene radical,
R7 is H or C₁-C₆-alkyl,
X and Y independently of one another can be a carbon atom which can be unsubstituted, monosubstituted or disubstituted by substituents inert under the reaction conditions, or a radical selected from the group consisting of N, O, S, or NR5R6, where R5 and R6 are as defined above, SO or SO₂,
n is 0,1,2 or 3,
Z is a carbon atom which can be unsubstituted, monosubstituted or disubstituted by substituents inert under the reaction conditions, or a radical selected from the group consisting of N, O, S, or NR5R6, where R5 and R6 are as defined above, SO or SO₂,
and at least one of the radicals X, Y and Z is not a carbon atom,
where the compounds of the formula (I), depending on the ring size, can have one or more double bonds in the ring, with the proviso that in the 5-membered ring the double bond is not conjugated with the -C(OH)CN-group,
and/or can be monoanellated or polyanellated by 5-membered, 6-membered or 7-membered rings containing 0 to 3 heteroatoms,
and/or can be bridged by an unbranched or branched C₁-C₆-alkylene radical, the alkyl chain of which can be interrupted by one or more heteroatoms and/or can have a double bond,
**characterized in that** ketones of the formula (II) are reacted, 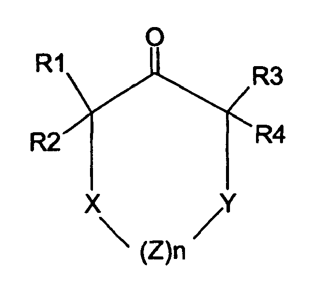 where R1, R2, R3, R4, X, Y, Z and n have the meanings given above, using an (R)- or (S)-hydroxynitrile lyase in the organic, aqueous or two-phase system or in emulsion in the presence of a cyanide-group donor to give the desired (R)- and (S)-cyanohydrins.

2. Process according to Claim 1, **characterized in that** ketones of the formula (II) are used as starting materials, where R1, R2, R3 and R4 independently of one another are H, a saturated or monounsaturated, unbranched, branched or cyclic C₁-C₁₂-alkyl radical in which a carbon atom in the chain can be replaced by an oxygen atom, a nitrogen atom or a sulphur atom, a phenyl, biphenyl or naphthyl radical, where the radicals can be substituted by one or more substituents selected from the group consisting of C₁-C₆-alkyl, C₁-C₆-alkoxy, C₁-C₁₂-alkylalkoxy, C₁-C₂₀-aryloxy, halogen, hydroxyl, oxo, carboxyl, phenyl which can be unsubstituted or monosubstituted or disubstituted by halogen, hydroxyl, C₁-C₄-alkyl or C₁-C₄-alkoxy,
or halogen, hydroxyl, oxo, C₁-C₆-carbalkoxy, C₁-C₆-carbalkoxyamino, NR5R6, acetyl, COOR7, cyano, amide, benzoylamino or NO₂,
R5 and R6 independently of one another can be H or an unbranched, cyclic or branched C₁-C₆-alkyl radical or together form a C₂₋C₈-alkylene radical,
R7 is H or C₁-C₆-alkyl,
n is 0, 1 or 2,
X, Y and Z independently of one another are a carbon atom which can be unsubstituted or monosubstituted or disubstituted by C₁-C₆-alkyl, C₁-C₆-alkoxy, C₁-C₁₂-alkylalkoxy, C₁-C₂₀-aryloxy halogen, hydroxyl, oxo, carboxyl, phenyl which can be unsubstituted or monosubstituted or disubstituted by halogen, hydroxyl, C₁-C₄-alkyl or C₁-C₄-alkoxy, or a radical selected from the group consisting of N, O, S or NR5R6, where one or two of the radicals X, Y or Z is not a carbon atom,
where the ketones of the formula (II) when n = 0 do not contain a double bond, when n = 1 do not contain a double bond, or contain one double bond and when n = 2 contain zero to two double bonds and/or can be anellated by a 5-membered or 6-membered ring containing 0 to 1 heteroatoms,
and/or can be be bridged by an unbranched or branched C₁-C₆-alkylene radical which can have a double bond.

3. Process according to Claim 1, **characterized in that** the organic diluents used for the reaction in the organic system are water-immiscible or slightly water-miscible aliphatic or aromatic hydrocarbons which may be halogenated, alcohols, ethers or esters or mixtures.

4. Process according to Claim 1, **characterized in that** the reaction is performed in emulsion.

5. Process according to Claim 1, **characterized in that** the enantioselective reaction is carried out in an aqueous system, where a solution or acetate buffer, borate buffer, phthalate buffer, citrate buffer or phosphate buffer solution containing the corresponding hydroxynitrile lyase or mixtures of these buffer solutions serves as reaction medium.

6. Process according to Claim 1, **characterized in that** the hydroxynitrile lyase used is a native or recombinant (R)- or (S)-hydroxynitrile lyase which is present either as such or immobilized.

7. Process according to Claim 6, **characterized in that** the hydroxynitrile lyase used is a native (S)-hydroxynitrile lyase from manioc or Hevea brasiliensis, recombinant (S)-hydroxynitrile lyase from genetically modified microorganisms selected from the group consisting of Pichia pastoris, E. coli or Saccharomyces cerevisiae, native (R)-hydroxynitrile lyases from Prunus amygdalus, Prunus laurocerasus or Prunus serotina, or recombinant (R)-hydroxynitrile lyases,

8. Process according to Claim 1, **characterized in that** the cyanide group donor is prussic acid, alkali metal cyanide or cyanohydrins of the general formula (III),
R7R8C(OH)(CN)
where R7 and R8 independently of one another are hydrogen or an unsubstituted hydrocarbon group, or R7 and R8 together form an alkylene group having 4 or 5 carbon atoms, where R7 and R8 are not simultaneously hydrogen.

9. Process according to Claim 1, further **characterized in that** the (R)- or (S)-cyanohydrins of the formula (I) are used for preparing the corresponding hydroxycarboxylic acids, ethers, esters or amides.

## Revendications

1. Procédé de préparation de (R)- et (S)-cyanhydrines hétérocycliques énantiomériquement enrichies, de la formule (I), 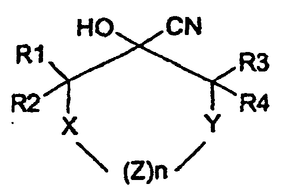 dans laquelle R1, R2, R3, R4 peuvent représenter indépendamment les uns des autres H ou un radical alkyle, alcényle ou alcynyle en C₁ à C₂₄ linéaire, ramifié ou cyclique, éventuellement substitué une ou plusieurs fois par des substituants inertes dans les conditions de réaction, un ou plusieurs atomes de C pouvant être remplacé(s) dans la chaîne par un atome d'oxygène, un atome d'azote, un atome de soufre, un groupe SO ou SO₂, un radical aryle ou hétéoaryle éventuellement substitué une ou plusieurs fois par des substituants inertes dans les conditions de réaction,
ou un radical hétérocyclique ou un halogène, un groupe hydroxy, NR5R6, acétyle, oxo, carbalcoxy en C₁ à C₆, carbalcoxyamino en C₁ à C₆, COOR7, cyano, amide, benzoylamino ou NO₂,
R5 et R6 représentent indépendamment l'un de l'autre H, un radical alkyle en C₁ à C₆ linéaire, ramifié ou cyclique, un radical phényle, un radical benzyle, COOR7, ou forment ensemble un radical alkylène ou hétéroalkylène en C₂ à C₈,
R7 représente H ou un alkyle en C₁ à C₆,
X et Y peuvent représenter indépendamment l'un de l'autre un atome de C éventuellement substitué une ou plusieurs fois par des substituants inertes dans les conditions de réaction ou un radical du groupe formé par N, O, S ou NR5R6, où R5 et R6 sont tels que définis ci-avant, SO ou SO₂,
n vaut 0, 1, 2 ou 3,
Z peut représenter un atome de C éventuellement substitué une ou deux fois par des substituants inertes dans les conditions de réaction, ou un radical du groupe formé par N, O, S ou NR5R6, où R5 et R6 sont tels que définis ci-avant, SO ou SO₂,
et où au moins l'un des radicaux X, Y et Z n'est pas un atome de C,
où les composés de la formule (I) peuvent, selon la taille du noyau, présenter une ou plusieurs doubles liaisons dans le noyau, à condition que, dans le noyau à cinq membres, la double liaison ne soit pas en conjugaison vis-à-vis du groupe -C(OH)CN,
et / ou être annélés une ou plusieurs fois par des noyaux à 5, 6 ou 7 membres comportant de 0 à 3 hétéroatomes,
et / ou être pontés par un radical alkylène linéaire ou ramifié en C₁ à C₆, qui peut être interrompu dans la chaîne alkyle par un ou plusieurs hétéroatomes et / ou présenter une double liaison,
**caractérisé en ce que** l'on fait réagir des cétones de la formule (II), 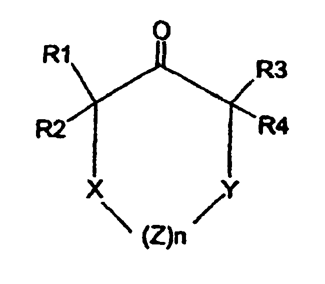 dans laquelle R1, R2, R3, R4, X, Y, Z et n ont la signification ci-avant, en présence d'un donneur de groupes cyanure, avec une (R)- ou (S)-hydroxynitrilelyase dans un système organique, aqueux ou biphasique, ou en émulsion, pour donner les (R)- et (S)-cyanhydrines souhaitées.

2. Procédé selon la revendication 1, **caractérisé en ce que** l'on met en oeuvre en tant qu'éduits des cétones de la formule (II), dans laquelle R1, R2, R3 et R4 représentent indépendamment les uns des autres H, un radical alkyle en C₁ à C₁₂ saturé ou à insaturation simple, linéaire, ramifié ou cyclique dans lequel un atome de C de la chaîne peut être remplacé par un atome d'oxygène, un atome d'azote ou un atome de soufre, un radical phényle, biphényle ou naphtyle, les radicaux pouvant être substitués par un ou plusieurs substituants du groupe formé par des radicaux alkyle en C₁ à C₆, alcoxy en C₁ à C₆, alkylalcoxy en C₁ à C₁₂, aryloxy en C₁ à C₂₀, halogène, hydroxy, oxo, carboxyle, phényle éventuellement substitué une ou deux fois par un halogène, un hydroxy, un alkyle en C₁ à C₄ ou un alcoxy en C₁ à C₄,
ou un halogène, un hydroxy, un oxo, un carbalcoxy en C₁ à C₆, un carbalcoxyamino en C₁ à C₆, NR5R6, un acétyle, COOR7, cyano, amide, benzoylamino ou NO₂,
R5 et R6 peuvent représenter indépendamment l'un de l'autre H ou un radical alkyle en C₁ à C₆ linéaire, cyclique ou ramifié ou former ensemble un radical alkylène en C₂ à C₈,
R7 représente H ou un radical alkyle en C₁ à C₆,
n vaut 0, 1 ou 2,
X, Y et Z représentent indépendamment les uns des autres un atome de C éventuellement substitué une ou deux fois par un radical alkyle en C₁ à C₆, alcoxy en C₁ à C₆, alkylalcoxy en C₁ à C₁₂, aryloxy en C₁ à C₂₀, halogène, hydroxy, oxo, carboxyle, un phényle éventuellement substitué une ou deux fois par un halogène, un hydroxy, un alkyle en C₁ à C₄ ou un alcoxy en C₁ à C₄, ou un radical du groupe formé par N, O, S ou NR5R6, où un ou deux des radicaux X, Y ou Z ne sont pas un atome de C,
où les cétones de la formule (II) ne contiennent pas de double liaison pour n = 0, ne contiennent pas ou contiennent une double liaison pour n = 1 et, pour n = 2, ne contiennent pas ou contiennent jusqu'à deux doubles liaisons et / ou sont éventuellement annélées par un noyau à 5 ou 6 membres comportant de 0 à 1 hétéroatome,
et / ou peuvent être pontées par un radical alkylène linéaire ou ramifié en C₁ à C₆, qui présente éventuellement une double liaison.

3. Procédé selon la revendication 1, **caractérisé en ce que** l'on met en oeuvre en tant qu'agent de dilution organique pour la réaction dans le système organique des hydrocarbures aliphatiques ou aromatiques non miscibles ou peu miscibles à l'eau, qui sont éventuellement halogénés, des alcools, des éthers ou des esters ou leurs mélanges.

4. Procédé selon la revendication 1, **caractérisé en ce que** la réaction a lieu en émulsion.

5. Procédé selon la revendication 1, **caractérisé en ce que** la réaction énantiosélective est entreprise dans le système aqueux, auquel cas l'une de la solution contenant les hydroxynitrile-lyases correspondantes ou de la solution de tampon acétate, de tampon borate, de tampon phtalate, de tampon citrate ou de tampon phosphate, ou des mélanges de ces solutions tampons, ser(ven)t de milieu de réaction.

6. Procédé selon la revendication 1, **caractérisé en ce que** l'on met en oeuvre en tant qu'hydroxynitrile-lyases des (R)- et (S)-hydroxynitrile-lyases natives ou recombinantes qui sont présentes telles quelles ou immobilisées.

7. Procédé selon la revendication 6, **caractérisé en ce que** l'on met en oeuvre en tant qu'hydroxynitrile-lyases des (S)-hydroxynitrile-lyases natives provenant de manioc et d'Hevea brasiliensis, des (S)-hydroxynitrile-lyases recombinantes provenant de microorganismes génétiquement modifiés du groupe formé par Pichia pastoris, E. coli ou Saccharomyces cerevisiae, des (R)-hydroxynitrile-lyases natives provenant de Prunus amygdalus, Prunus laurocerasus ou Prunus serotina ou des (R)-hydroxynitrile-lyases recombinantes.

8. Procédé selon la revendication 1, **caractérisé en ce que** l'on met en oeuvre en tant que donneurs de groupes cyanure de l'acide cyanhydrique, des cyanures de métaux alcalins ou des cyanhydrines de la formule générale (III),
**R7R8C(OH)(CN)**
dans laquelle R7 et R8 représentent indépendamment l'un de l'autre de l'hydrogène ou un groupe hydrocarboné non substitué, ou bien R7 et R8 forment ensemble un groupe alkylène comportant de 4 à 5 atomes de C, où R7 et R8 ne représentent pas simultanément de l'hydrogène.

9. Utilisation selon la revendication 1, **caractérisé en outre en ce que** les (R)- ou (S)-cyanhydrines de la formule (I) sont utilisées pour la préparation des acides hydroxycarboxyliques, éthers, esters ou amides correspondants.
